# EUROPEAN PATENT APPLICATION

(11) **EP 2 298 241 A2**
(43) Date of publication of application: **23.03.2011**
(21) Application number: 10012231.6
(22) Date of filing: 03.12.1997
(51) Int. Cl.: A61F 2/06

(54) **Multi-stage prothesis**

(30) Priority: 03.12.1996 US 759877; 03.12.1996 US 760113; 03.12.1996 US 760115
(62) Divisional of application: 97954565.4
(71) Applicant: Atrium Medical Corporation, Hudson, NH 03051 (US)
(72) Inventor: Karwoski, Theodorc, Hollis, NH 03049 (US); Gingras, Peter, Belford, MA 01730 (US); Markatos, Paul, Pelham,NH 03076 (US); Herweck, Steve A., Nashua, NH 03062 (US)
(74) Representative: Helbig, Christian

(57) **Abstract**

The invention relates to an improved tubular prosthesis for surgical implantation to replace a segment of a blood vessel, a method of forming a tubular prosthesis and an endostent for insertion into a biological flow passage.

## Description

### Field of the Invention

This invention relates to a porous polytetrafluoroethylene structure that can be formed into an implanted prosthesis with improved physical strength and surgical handling for various applications. In different embodiments, the invention is adapted to form structures as various as endovascular liners or supports and prosthetic vessels wherein the entire unit has a high degree of tissue compatibility and structural integrity. These constructions enjoy enhanced physical properties, such as kink and compression resistance, ease of tunneling during surgical placement, resistance to natural dilation and physical strength degradation in arteriovenous applications, or alternatively, adaptability to mechanical dilation with a high degree of dimensional stability and strength thereafter. The invention also relates to methods of manufacture of the prostheses.

This invention also relates to a lamellate polytetrafluoroethylene material that can be formed into an implant where there is an improvement in the surgical handling accompanied with enhanced healing properties due to the novel arrangement of variable porosity regions of polytetrafluoroethylene. This invention relates to materials utilized in the production of devices for in vivo implantation, such as heart valve leaflets, sutures, vascular access devices or any related products, but more particularly relates to vascular grafts, for example, to porous polytetrafluoroethylene prostheses intended for placement or implantation to supplement or replace a segment of a natural, biological blood vessel. It also relates to patches or supports for tissue repair or reinforcement. For simplicity of exposition below, the invention will be discussed solely with relation to an implantable vascular graft, or a liner for a vessel which might, for example, be delivered intraluminally.

The present invention also relates to vessels and vascular support structures, such as stays, stents and support rings which are used for maintaining open a biological passage, such as an artery.

### Description of the Prior Art

Conventional vascular grafts manufactured from porous polytetrafluoroethylene have limitations in surgical handling and healing. In some instances, the porous grafts are wrapped with an external reinforcing film to increase radial strength. Vascular grafts may also be reinforced with an external spiral bead or ring. The reinforcing film does not provide radial support to prevent kinking and collapse during placement or during access use. Furthermore, the presence of an external bead or ring results in interference during surgical placement increasing trauma to the surrounding tissue. In addition, such grafts may be stiff and noncompliant to the natural artery.

Surgical implantation procedures require placement of the vascular graft within the subcutaneous tissue of humans. Peripheral and angioaccess vascular procedures require an anatomic or subcutaneous pathway commonly called tunneling. Tunneling is an initial surgical step in the vascular procedure which can result in localized injury to adjacent tissue. The tunnel diameter relative to the implant diameter, as well as the abrasive force exerted by the implant to the adjacent tissue have a significant impact on the resultant healing response.

It is advantageous in the clinical setting to minimize trauma through ease of tunneling. One approach is to use an expensive surgical tool that often results in larger than required pathways influencing the healing response by creating a fibrous capsule that surrounds a fluid sac that does not incorporate the implant.

One problem which can arise with current PTFE arteriovenous grafts is a lifespan limitation due to physical attrition of the graft caused by poor dialysis access technique identified by repeated needle punctures in concentrated areas resulting in ever enlarging holes or tears in the material comprising the graft wall. Maturation of the surrounding tissue incorporating a vascular access graft, to reduce the adventitial space between tunnel and implant, is a prerequisite to use of the graft for subsequent use in dialysis. The maturation time is necessary to prevent tunnel hematomas which can occur from premature graft puncture. For this reason, it is currently recommended that one to four weeks pass before initial needle puncture is performed.

Some known constructions incorporating PTFE as the sole or a large portion of a vascular graft include constructions wherein an inner tube is surrounded by one or more other layers of tubing, foam or fiber wrapping to enhance its mechanical compliance and, for example, provide direct impermeability, or result in clotting which, after a short time, becomes impermeable. The inner tube is generally formed of PTFE, selected for its highly advantageous biocompatibility properties in the blood path. Various outer layers may consist of fibers either helically wound or electrostatically flocced, films of thin material, tape wrap generally also of thin material, or coatings. Materials used for these layers may also include impermeable polyurethane or other soluble polymer coatings, emulsions and also PTFE films.

These composite structures are in some ways similar to the earlier generation of fabric grafts made of woven or knitted Dacron or the like, and each represents an attempt to address or optimize some of the various constraints encountered in trying to replace a vessel with material which is strong, capable of long term patency and has some degree of tissue compatibility.

In general, however, conventional vascular grafts manufactured from porous polytetrafluoroethylene have limitations in surgical handling and healing.

Presently, many vascular grafts exhibit some degree of weeping or blood loss during implantation. A variety of factors effect this surgical complication, one being prewetting of vascular grafts with heparinized saline or antibiotics to render the surface thrombus and infection resistant. Prewetting of the graft results in a reduction of the hydrophobic properties with an effective increase in permeability. Cohesion of platelets and adhesion of fibrin in the graft wall can initiate the coagulation cascade resulting in thrombus formation. The thrombi are responsible for the formation of emboli in tubular prosthesis with small diameters.

Native arteries and veins have a common pattern of organization made up of three layers: an internal intima, surrounded by a media, and then an external adventitia. Each of these layers has a predominant structure and cell-type. The walls of arteries are built of elastin, collagen, a non-fibrous glucosaminoglycan-rich matrix and smooth muscle cells. The microscopic structure of the artery wall correlates with the function of the various wall-layers and components.

Several studies support the belief that there is a net transport of macromolecules across the arterial wall. The transport process is controlled by diffusion, convection, and other forces. Convection is associated with the hydraulic flux resulting from pressure or osmotic differences across the arterial wall. Diffusion occurs in response to a concentration driving force.

A great many constructions for both reinforced prostheses and separately-applied stents are known in the art, ranging from simple wire or plastic rings and arrangements of stiff but flexible sheets or shells, to technologically advanced constructions wherein a wire structure of heat memory alloy flips to an enlarged memory configuration, or wherein a solid tubular body is fabricated with microscopically thin laser-cut slots which convert the solid cylinder into an expandable body that opens out to form a mesh-like but reasonably stiff surface support. One commercial embodiment of this latter type of stent, referred to as the Palmaz stent after a surgeon who popularized this construction, is in common use now. Another common form of stent consists of wire crimped into a zig zag pattern which can be expanded to attain a much larger length or diameter. Stents of this form may be formed as individual rings, or serpentine windings, or as pairs of helical windings which act against each other to counterbalance twist while expanding radially. Numerous other constructions are known.

Many if not all of the materials used for stents involve metal or carbon fiber materials which are highly electro-positive and are bio-active. Since stents tend to be used under conditions where they are counteracting disease processes, supporting healing processes, or guarding against stenosis of a passage, bio-activity, which may encourage undesirable or poorly regulated growth processes, or lead to clot formation, should be avoided. Coating of the stent can keep the stent from directly contacting surrounding tissue or fluids, and thus can theoretically protect against unwanted electrochemically induced tissue reactions.

In the field of expandable stents, however, a further problem arises due to the fact that many effective or compact stent constructions involve filamentous or wire-like structures which have numerous apertures or spaces between the various strands or structural elements of the stent. With these constructions, tissue may grow through the openings of the stent. Furthermore, the stent itself may provoke a foreign body reaction and be both a stimulus for and a framework supporting, proliferative tissue growth, resulting, for example, in scar tissue or restenosis of the very region it is placed to control.

One approach to this drawback is to provide a coating, liner or cover for the stent which prevents the healing or diseased layer of tissue from directly contacting the stent or from passing through the stent in any way. Such liners may be formed, for example, of porous polytetrafluoroethylene (PTFE) which allows the passage of fluids and vital materials while serving as a barrier to tissue growth. However, when applying such a construction, a further difficulty which may arise is that the layer or sleeve of polymer must be attached to the stent for example, by staples or sutures at one end, or is prone to developing loose pockets or folds which might accumulate organic matter or lead to sepsis or unusual growth. Also, the necessarily thin liner material may detach or degrade. The risk of loose or unattached liner material is particularly great for constructions which utilize poorly adherent polymers, such as PTFE, or structures which seek to combine an expandable stent of stiff material, which changes both its dimension and its shape, with a dissimilar liner or shell.

Accordingly, there remains a need for a covered support construction of enhanced hardiness and implant compatibility.

There is also a need for an expandable vessel support which forms a unitized and non-delaminating tissue barrier.

There is also a need for a need for a vascular liner having atraumatic properties and haemodynamic shape.

While a number of vascular grafts, or processes for preparing the same, provide for a stronger graft, such grafts do not generally possess a differential permeability effective to achieve enhanced healing and tissue ingrowth, and at the same time offer improved surgical handling.

There is a need for an in vivo implantable material prosthesis, and in particular vascular grafts which are formed as a lamellate structure that mimics the natural artery with differential cross-section permeability composed of collagen and elastin and is acceptable to the surrounding tissue.

There is also a need for an implantable vascular device having improved physical strength for cannulization.

It also remains desirable to provide prostheses or material having enhanced tissue compatibility or long term patency or growth compatibility characteristics.

### Summary of the Invention

Broadly speaking, the present invention provides for an implantable multistage structure which has an integrated wall structure substantially comprised of porous fluoropolymer material.

In one aspect the article has an integral reinforcement within the device wall, and has that allow for improved surgical handling at implantation, reduced tissue trauma to provide improved healing, and improved performance in an arteriovenous device, together with a method for making the same.

The implantable multistage PTFE porous structure of the invention includes an integral circumferential support within the cross-section with one or more thickness zones within the cross-section having smaller than average pore diameter than the other sections, and in which all the zones have been bonded to the adjacent zones completely throughout the interfaces, free of interlaminar peeling.

The multi-stage structure may be in the shape of any suitable medical implantable device. However, the structure of the invention is particularly advantageous when in the form of an implantable tubular prosthesis, such as a vascular graft.

One embodiment of the present invention includes in vivo implantable structures formed with a two or more zones of different node/fibril geometry with an integral intrazone circumferential support. An object of this invention is to provide shaped products manufactured from PTFE that are biologically compatible with surrounding tissue. Another object of the present invention is to provide an in vivo implantable material having improved surgical handling and implant performance.

The biologically compatible material of the present invention has excellent compatibility, strength, and surgical handling because of the arrangement of integral support and node/fibril PTFE fibrous structures. Some current vascular prostheses are designed with an external biaxially oriented reinforcement wrap, spiral bead, or ring, in direct contact with adjacent tissue, to provide additional radial strength to a tubular product, but which results in poor surgical handling during placement and poor compliance. Tubes of the present invention provide improved surgical handling during placement which results in quick maturation and tissue incorporation leading to good healing. In addition, tubes of the present invention provide for greater needle holes per unit area without physical strength compromise in order to address the problem of premature physical failure due to poor cannulation technique.

The products of the present invention have a very broad application in medical devices, such as vascular grafts, endovascular devices, and vascular access devices. In a preferred embodiment, each radial cross-section region of the implant can be distinguished from other regions by having different pore size, pore shape, and porosity in conjunction with an intrawall circumferential support integral to the structure. Indeed, the fibril-nodal microstructure throughout the matrix may have the internodal distance, i.e. pore size, in one section at least two to twenty times that for its adjacent sections. One in vivo material has two cross-section regions. The first region, for example, has an internodal distance of the pores of the PTFE luminal surface of about 20 or 30 microns and a specific node/fibril geometry. In the next zone the internodal distance of the pores is a range from about 1 to about 10 microns and a specific node/fibril geometry, preferably 1 to 5 microns. This pore size is excellent for cell growth mediator permeability, instead of undesired encapsulation. Another embodiment of the present invention includes the luminal surface and second and third zones of material previously described whereby the third zone has a pore size range of 50 to 500 microns and a specific node/fibril geometry, preferably about 50 to 100 microns which is excellent for fibroblast tissue ingrowth, as the healing process progresses. In a further embodiment, a circumferential support having a radius of diameter from 25 to 1000 microns is present within the wall structure to provide kink and compression resistance along with dialysis technique improvement.

As discussed above, one embodiment of the present invention includes an in-vivo implantable material comprising the luminal, second, and third regions in combination with an integral circumferential support previously described. Another embodiment of the present invention includes the luminal, second and third region of material previously described with the third region or the integral support providing a source location for drug delivery.

In a still further preferred embodiment of this invention, a fluoropolymer bead is wrapped around the outer surface of the composite structure under tension. This embodiment is particularly useful in the preparation of vascular grafts. That is, the multistage structure is a tubular shaped structure with maximum compression resistance having particular utility in applications where such properties are extremely advantageous, (i.e., peripheral bypass surgery, endoluminal).

The above described devices do not have to be totally implanted within the body to be considered within the scope of the present invention and include, among other devices, catheters, transcutaneous tubing or artificial skin.

In accordance with another aspect of the present invention there is provided an implantable or prosthetic material with at least first, second and third regions through the wall thickness extending continuously along the length and width thereof, and wherein material of the innermost and outermost regions has a cellular compatibility property such as node size or reticulation structure, while at least one, preferably an interior, region of the wall modulates hydraulic pressure otherwise passing through the porosity of the prosthesis. The first, second and third regions join or merge continuously together along their bounding surfaces, and form a unitary or integrated wall body.

The products comprising this aspect of the instant invention have a very broad application in medical devices, such as vascular grafts, endovascular devices, vascular access devices, transcutaneous access devices, synthetic heart valve leaflets, artificial organ implants, etc. In a preferred embodiment, each cross-section region of the implant can be distinguished from other regions by having different pore size, pore shape and porosity. Indeed, the fibril-nodal microstructure throughout the matrix may have the internodal distance, i.e, pore size, in one section at least two to twenty times that for its adjacent sections. An *in vivo* material having three cross-section regions, for example, the internodal distance of the pores of luminal surface of PTFE vascular graft is about 20 or 30 microns with a corresponding WEP of 200 mm Hg and a specific node/fibril geometry. The internodal distance of the poces of the next zone comprise a range from about 1 to about 10 microns with a corresponding WEP of 400 mm Hg or greater and a specific node/fibril geometry, preferably about 5 to 10 microns. The pore size is excellent for cell growth mediator permeability, as distinguished, for example from total impermeability which causes an undesirable state of encapsulation. Another embodiment of the present invention includes a luminal, second, and third zone of material as previously described whereby the third zone has a pore size range of 50 to 500 microns with a corresponding WEP of 100 mm Hg or less and a specific node/fibril geometry, preferably with an internodal distance of about 50 to 100, to effectively promote fibroblast tissue ingrowth, as the healing process progresses. The lamellate structure of the present invention offers a wall architecture similar in nature to that of a native vessel which contains an intima, media, and adventitia.

A further embodiment of the present invention includes *in vivo* implantable material as described above in the form of a sheet, tube or enclosure comprising a luminal, a second and a third region as previously described. Another embodiment of the present invention includes the luminal, second and third region of material as previously described with the third region being filled to provide a source location for drug delivery.

For a vascular prosthesis, the outer wall may have a porosity or regular structure of channels which is compatible with and serves as a microscaffolding structure for the growth of connective tissue. The inner face of the prosthesis on the other hand may have a smaller pore structure, optimized for attachment of a neointima for reconstituting a natural biological flow surface at the interior of the vessel. The modulation region serves the function of blocking the direct or immediate transmission of hydrostatic pressure or fluid migration through the thickness dimension of the wall, and prevents through-growth of tissue, allowing a stratification of tissue layers to redevelop over time in a more natural fashion after the prosthesis has been implanted. Pore structure of the modulation region may be irregular, and generally is either small in size, or tortuous in path. The modulation region may also have non-existent porosity, i.e., be a continuous solid.

The prosthesis may be constructed from plural layers or tubes of material by radially nesting a first, second and third layer of material, either as tubes, wound sheets or a wrap and then coalescing the three separate bodies together into a continuous wall body in which each region through the thickness retains the structure of the starting material for that region. Preferably, the entire structure is made from PTFE, or PTFE with another fluoropolymer.

In accordance with yet another aspect of the present invention, a radially expandable support body is enclosed within a solid but expandable polymer body of porous and expanded PTFE material that physically isolates the support body from surrounding blood and tissue while providing an protective surrounding that retains its integrity upon expansion.

In one preferred construction, the support body is a stent that is cocooned within a cuffed sheet. In this construction, the sheet is originally a tube of polytetrafluoroethylene (PTFE) material, which passes through the interior of the stent and is cuffed, e.g., is folded back upon itself, over the stent, in a manner similar to the folding of a sock, so that the folded-back end of the tube becomes an outer layer smoothly extending around the end and covering the outside of the stent. The assembly is then heated, causing the outer layer to shrink and coalesce with the inner layer so that the stent is enclosed within a folded envelope having a continuous and seamless end portion. Preferably, radial pressure is applied during the heating so that the layers conform tightly to the support body and fill all interstitial spaces thereof. In other constructions, support members lie within pockets extending in the direction of expansile deformation.

Preferably, the tube is porous PTFE, having a microstructure of fibrous material interconnecting nodes of solid polymer, and the PTFE forms a soft and pliant surface that cushions the edges of the support body, or stent, and blocks direct contact between the stent and surrounding tissue, so that any fluids or material must penetrate the mat of fibrils to contact the stent environment. By first expanding an end portion of the tube before folding it back over the stent, the end portion, which becomes an exterior surface of the finished product, may be provided with a degree of porosity which is greater than that of the interior surface. In a further embodiment, each end of the central tube is so expanded, and then folded back so that the assembly is closed over at both ends and has a single seam extending circumferentially around the outside where one end meets or overlaps a portion of the other end of the tube part way along the body of the assembly. Alternatively, the outer surface may be covered by a wrap, or by a separate polymer tube; in this case the inner tube may have a relatively short end cuff portion, which is preferably folded over the outer cover for a short distance.

In a preferred embodiment, the entire inner and outer portions are formed of a single PTFE tube and are heated to both shrink the tube down into a compact and thin film-like cocoon, and to coalesce the inner and outer layers together at all points where they come in contact so that the polymer cocoon becomes unitary and non-delaminating. Preferably, the stent body itself is of limited axial extent, like a ring, or a series of spaced-apart rings, or else it possesses a number of apertures extending entirely through the stent at short axial spacing, so that the remaining spaces or apertures are covered over or bridged by both the inner and outer polymer layers, which coalesce into a continuous barrier. The apertures, which may comprise five to eighty percent or more of the surface area of the stent, constitute a grid or network of regions or tack points through which the material is coalesced and continuously bonded. When the stent is expanded, its changes in dimension and orientation may locally introduce shear which separates the stent or support body from the polymer. However, the support body is able to shift only within the regions where the inner and outer portions of the tube have not coalesced to each other, and thus it locally distributes strain to the surrounding polymer in a manner generally effective to prevent rupture and prevents the development of extended pockets or voids which could impair performance in use.

In embodiments where a two tube construction is cuffed and assembled to arrive at a similarly unitized and seamless stent. This is done as follows: first a tube of polymer is placed through the center of the stent and the ends of the tube are folded back over the stent for a short distance, or are expanded in radial extent and then folded back. Next, a second tube is placed over the outside of the stent covering the folded back ends of the first tube. As before, the assembly is then passed through an oven to shrink the outside and inside layers into a unitary coalesced covering enclosing the entire stent, which is continuous and seamless over the end regions. One variation of this two-tube construction is to place the second tube over the stent before folding back the cuffs formed by the inner tube. In that case, the cuffs cover the ends of the second tube. The second tube may be a tube having different porosity than the first tube and may for example, have the node size of twenty to one hundred micrometers or more, and preferably thirty to sixty micrometers, which is suitable for ingrowth of surrounding tissue. This serves to better anchor the structure in the stented passage. Alternatively, both inside and outside polymer walls may have a relatively small pore size of one to five micrometers to provide a higher degree of isolation of the stent from surrounding tissue, or assure that tissue does not penetrate through the continuous boundary.

### Brief Description of the Drawings

These and other features of the invention and its various aspects and embodiments will be understood from the description herein, taken together with illustrative figures illustrating constructions and representative examples thereof, wherein:
Figure 1A-1D is a schematic illustration of a process for manufacturing a tubular prosthesis in accordance with the principles of this invention;
Figures 2A and 2B are microphotographs of a wall cross section of two embodiments of an implantable prosthesis constructed in accordance with the principles of this invention;
Figure 3A is a cross-sectional image through a wall of a first embodiment of multilamellate prosthesis of the invention;
Figure 3B is a cross-sectional image through a wall of a second such embodiment of the invention;
Figure 3C is a cross-sectional image through a wall of a third such embodiment of the invention;
Figure 4 is a cross-sectional image through a wall of a fourth embodiment of the invention;
Figure 5 is a cross-sectional image through a wall of a fifth embodiment of the invention;
Figure 6 is a cross-sectional image through a wall of a sixth embodiment of the invention;
Figure 7 is a cross-sectional image through a wall of a seventh embodiment of the invention;
Figure 8 is a cross-sectional image through a wall of a eighth embodiment of the invention;
Figure 9 is a cross-sectional image through the wall of a ninth embodiment of the invention;
Figure 10 shows a vascular prosthesis according to any of the above embodiments of the present invention;
Figures 11A-11C schematically show a method of forming the enclosed stent of the present invention;
Figures 12A-12D illustrate a two-element unitized stent construction;
Figures 13A-13E illustrate another two element unitized construction and the method of making it;
Figures 14A-14C show an embodiment with covered ends and a seamless interior and a method for making it; and
Figures 15A-15C show several non-uniform expansion embodiments.

### Detailed Description

Expansion of extruded PTFE material is generally known in the art. The structure obtained is a direct result of extrusion and expansion conditions. For example, extrusion variables such as resin type, lubricant levels within the preform, and reduction ratio will have a significant effect on post extrusion processed material. Expansion conditions play a role whereby, in general, material expanded at lower temperatures and faster rates will possess a finer node/fibril structure with higher water entry pressure (WEP) and longitudinal tensile strength (LTS); compared to material expanded at higher temperatures and lower rates which has a coarser node/fibril structure possessing lower WEP, higher radial strength (RBT, RTS), and increased suture strength (SRT).

A PTFE porous tube which can be used in the present invention may be initially produced by a method which is basically the same as the one described in United States Patents No. 5,433,909 and No. 5,474,824. The method comprises the step in which a mixture of unsintered PTFE powder and a liquid lubricant is supplied into a ram extruder to extrude in a tubular form, the tube thus obtained is then stretched in the longitudinal direction, while the liquid lubricant is or is not removed from the tube; thereafter while the stretched tube is fixed to prevent shrinkage, the stretched tube is sintered by heating to a sintering temperature of 327°C or more to fix the stretched structure. The resulting PTFE porous tube provided has a microfibrous structure comprising nodes interconnected with fibrils. The diameter and length of the fibrils and the size and number of the nodes can be varied by changing the conditions of stretching operations, and thus the pore size and porosity of the porous tube thus obtained can be freely controlled. The foregoing patents describe methods of making extruded PTFE material having large oriented nodes, uniaxially oriented fibrils and a pore structure of oriented channels, and they describe methods of controlling the size and spacing of the node-defined radially extending through channels. They further describe methods of manufacturing tubes and prosthetic material such that this pore structure may differ at different surfaces, or vary along the thickness dimension of the material. Each of the aforementioned United States Patents is hereby incorporated by reference herein in its entirety

As illustrated in the drawing, the structure contemplated by the present invention may be attained by the following procedures. Various porosities of PTFE in a tubular form having a predetermined inner diameter are radially expanded to a size larger than the original diameter, placed on a stainless steel forming mandrel, circumferentially supported with an integral support, and formed to the final configuration, by heating to a temperature of 327°C or higher until it acquires a multi-stage structure. By this process, the integral support is located between both surfaces of the tube and within the fibrous structure of PTFE. The present invention offers this PTFE porous tube as a tubular prosthesis.

As described above, by appropriately controlling the temperature and time conditions to be employed for stretching operations, along with the arrangement of zones within the wall cross-section, the PTFE tube can be provided with a profile of gradual change in its fibrous structure through the thickness of the tube wall wherein the porous structure of the inner surface is separated from the outside surface.

In a porous, fibrous material, that part of the total porosity which is available to fluid flow is called the "effective porosity". The pressure required to force a liquid into a pore is a function of pore size and geometry, liquid surface tension, and solid/liquid contact angle. Surface tension opposes the entry of any nonwetting liquid (any liquid having a contact angle with surface of the material greater than 90°) into a pore and this opposition may be overcome by external pressure.

In material science, there is a distinction between material porosity and permeability. Porosity is a direct measure of the physical void volume contained within a boundary, whereas permeability refers to the accessibility of that void volume. Permeability is usually expressed as a rate of flow of liquid or gas per unit area, as a function of differential pressure.

Permeability to fluid flow can be determined by measuring the amount of pressure required for water to permeate the pores of the material. To compute water entry pressure (WEP) one subjects the material to an incrementally increasing water pressure until small beads of water appear on the surface. WEP is a gage which can be used to equate porosity to permeability.

Vascular graft porosity is a measure of the void fraction within the prosthesis wall and is believed to give a rough prediction of the capacity of the graft to anchor newly formed surrounding tissue after implantation, whereas permeability is associated with fluid flow through the graft wall.

Vascular permeability or hydraulic conductivity is related to material porosity. Water entry pressure (WEP) is a good measuring technique in this application because it closely mimics the permeation process at the blood/prosthesis interface. WEP is defined as the pressure value necessary to push water into the pores of a synthetic tubular substrate and can be classified as: High 400 mm Hg), Medium (200-400 mm Hg), and Low (<200 mm Hg).

It has been widely accepted since the nineteenth century that the hydrostatic pressure difference across the arterial wall is capable of transporting water from the blood into the surrounding interstitial space. The filtration coefficients of the wall are dependent on the hydraulic conductivity of both the intima and media. The artery wall is a heterogeneous porous medium in which interstitial fluid can flow through the interstices between cells and tissue mimicking a semipermeable membrane with hydrostatic and osmotic pressure components. The osmotic pressure difference across the vessel wall is assumed to be small compared with the hydrostatic pressure or hydraulic conductivity.

Expanded PTFE material is characterized by lengthwise-oriented fibrils interrupted by transverse nodes. The pore size in microns is typically determined by measuring fiber length between the nodes (internodal distance). To compute fibril length, the material is viewed under sufficient magnification. A fibril length is measured from one edge of one node to the edge of an adjacent node. Fibril lengths are measured from the sample to compute a statistically significant mean fibril length.

Nodes and fibrils may be further characterized by their relative geometry. That is, nodes by length, width, and height; and fibrils, by diameter and length. It is the relative geometry of nodes to fibrils, as well as, internodal distance that determines porosity and permeability of porous PTFE.

In accordance with a basic aspect of the present invention, a prosthetic structure is formed wherein inside and outside surfaces of the structure have controlled characteristics while an overall physical characteristic of the device, such as radial strength, cannulizability, hydrostatic gradient control or other such feature is tailored or controlled by an inner portion. Elements of the fabrication methods involved are best illustrated by a first aspect of the invention wherein a winding or bead provides elements of strength and improved handling in a vascular graft.

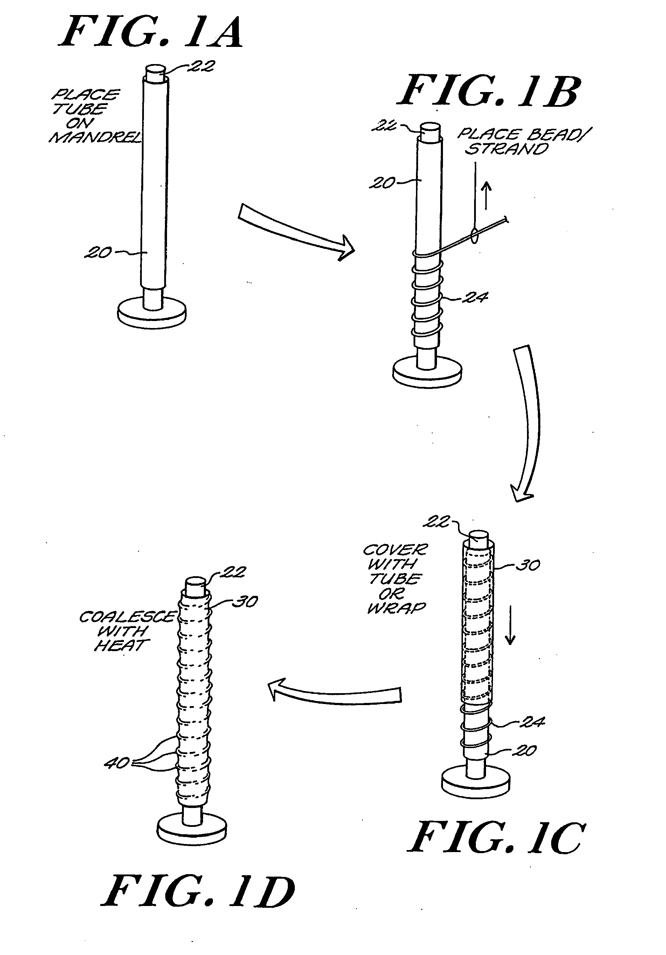

As illustrated in Fig. 1A through 1C, the process may be considered in four discrete steps. In step one (Fig. 1A), a tube 20 formed of PTFE resin is placed on a tight-fitting stainless steel forming mandrel 22. The tube 20 may be formed from PTFE resin (Fluon CD-123 obtained from ICI Americas) which has been blended with 100 grams of "Isopar H" odorless solvent (produced by Exxon Corporation) per pound of PTFE, compressed into a preform billet and extruded into a 6.0 mm I.D. and 6.8 mm O.D. tube in a ram extruder having a reduction ratio of about 200:1 in cross-sectional area from billet to extruded tube. After removal of lubricant, the extruded tube is expanded and sintered, according to the method described in the aforesaid US Patents under various conditions to produce material with different node/fibril structures.

In the next step (Fig. 1B), a bead of diameter less than 1 mm., for example, a 375 micron diameter PTFE bead 24 may be wrapped circumferentially in a helical manner around the tube 20. In a third step (Fig. 1C) a PTFE outer tube or wrap 30 covers the tube 20 with its helically wrapped beads. This tube 30 may be formed using PTFE resin (FLUON CD-123 obtained from ICI Americas) blended with 100 grams of "Isopar H" odorless solvent (produced by Exxon Corporation) per pound of PTFE, compressed into a preform billet and extruded into a 2.0 mm I.D. and 2.4 mm O.D. tube in a ram extruder having a reduction ratio of about 200:1 in cross-sectional area from billet to extruded tube. After removal of lubricant, the extruded tube was expanded and sintered, according to the method described in the aforesaid US Patents incorporated herein for reference, under various conditions to produce material with different node/fibril structures. This tube 30 is dilated to an 8 mm O.D. prior to placing it over the beaded tube 20.

In the final step (Fig. 1D), the outer tube 30 is restrained to prevent longitudinal shrinkage and is then transferred to an oven at 360°C for 5 minutes to coalesce the inner and outer tubes 20 and 30 respectively, thereby enclosing and smoothly covering ridges 40, to provide the final structure.

The helical bead 24 is wrapped around tube 20 with a pitch such that the spaced apart protruding ridges 40 are spaced at a distance, such as to 1-3 mm, which is effective to trap a needle inserted into said space thereby preventing longitudinal tearing of the prosthesis when cannulized with a dialysis needle.

In an alternative method the first tube 20 is formed of PTFE resin (Fluon CD-123 obtained from ICI Americas) blended with 100 grams of "Isopar H" odorless solvent (produced by Exxon Corporation) per pound of PTFE, compressed into a preform billet, extruded into a 4.0 mm I.D. and 4.6 mm O.D. tube in a ram extruder and having a reduction ratio of about 200:1 in cross-sectional area from billet to extruded tube. After removal of lubricant, the extruded tube is expanded and sintered, according to the method described in the aforesaid US Patents incorporated herein for reference, under various conditions to produce material with different node/fibril structures. The PTFE bead 24 is extruded to a 250 micron diameter, and is circumferentially wrapped in a helical manner. Thereafter an outer tube 30 formed as in the first process is dilated to a 6 mm O.D. and then, as in the prior process embodiment, is heated to coalesce the tubes to form a multistage structure.

In a third process variation the beading 24 may be formed as a metal wire core enveloped by a PTFE jacket.

In a fourth alternate process, rather than a helical winding, discrete bead rings at an axial spacing between one and five millimeters form a segmented supporting structure.

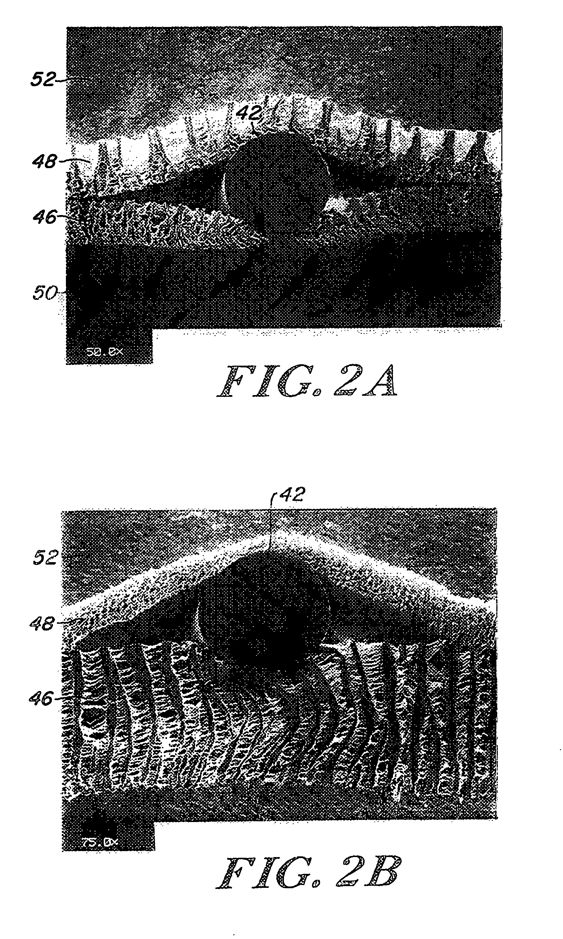

With reference now to Figs. 2A and 2B, microphotographs at a magnification of 50X and 75X, respectively, of the cross section of a prosthesis wall of two embodiments of a product produced by the above described method are shown. With reference to Fig. 2A, the inner, or luminal, surface 46 of a prosthesis wall is formed of a PTFE material characterized by a relatively low density, and a porosity having relatively large pores interconnected by fibrils. Wrapped around that surface is a bead 46 which as above described can be formed either of a solid PTFE, or by a wire or metal core covered by PTFE. The next zone of the wall is a wrap cover 48 of PTFE which has been coalesced by heat to envelope both the inner surface 46 and the bead 42. In some embodiments the porosity of the cover 48 may be (as illustrated in Fig. 2A) a different porosity than that of the inner surface 46. Finally the outer surface of the prosthesis wall 52 may again be formed of a relatively low porosity PTFE material.

Fig. 2B shows a similar structure wherein the porosity of the inner, luminal zone 46 is greater than that of the wrap cover 48. In each case, the article is assembled and coalesced into a unitary structure such that the inner and outer surfaces are presented for biocontact when implanted, while the bead remains enclosed and to some extent cushioned and immobilized by the surrounding surface portions.

In addition to articles having an inner bead or winding, the present invention contemplates articles wherein the inner structure forms a layer or sheet of substantiallly homogeneous properties in two dimensions, such as a sheet or tube of a defined pore structure, or a stent or series of stents, and wherein the property of the inner portion produces, controls or modulates the desired physical characteristic.

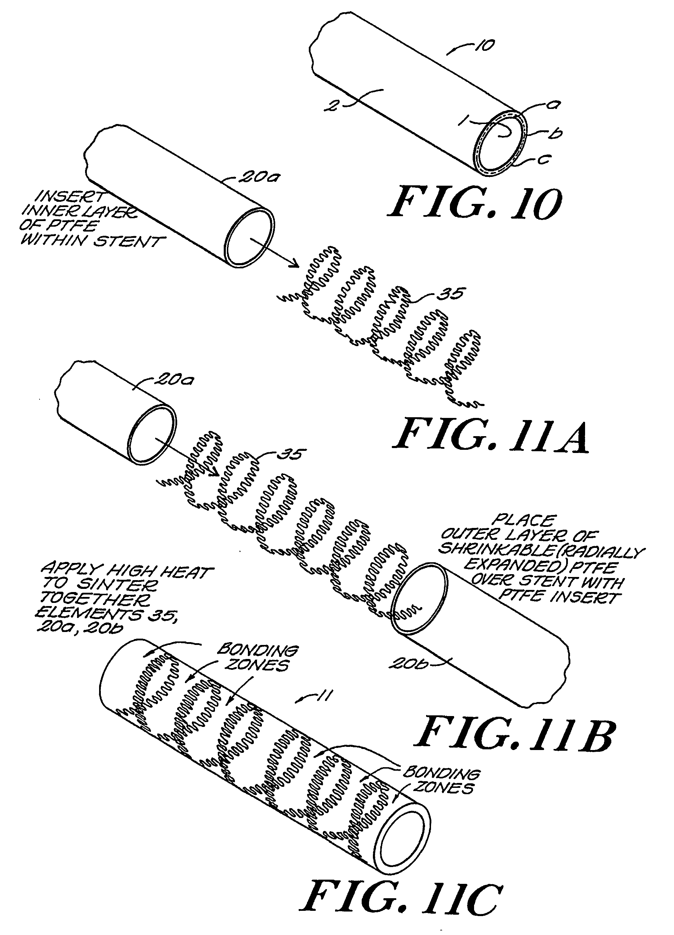

Figure 10 illustrates an implantable prosthetic member 10 according to this aspect of the present invention, which, is shown in the figure as a tubular member, suitable for implantation as a vascular graft. The member 10 has an inner wall 1 and an outer wall 2 with a thickness dimension extending therebetween. As further illustrated in Figure 10, there are at least three continuous regions adjacent to each other and extending along the entire area of the member namely, regions a, b and c, illustratively shown in the Figure as concentric strata from the inside to the outside. As described in more detail below, the successive regions a, b, c are not separate structures but are portions of the same wall, and are distinguished by their structural properties as relates in particular to aspects of porosity.

In general, each embodiment of the invention includes at least one region having a zero or sufficiently low porosity that it effectively acts as a barrier to fluid penetration or a barrier which modulates transmission of hydraulic pressure pulsation through the thin wall of the prosthesis. This barrier region may be a completely pore-free stratum, a stratum having small pore size, or a stratum having a high density of crossed, irregular, dead end, or closed cell pores such that it carries out its modulation or barrier function. In the latter case, even large pore material may be used, but its water entry pressure (WEP) is high. This stratum may exist at the region of inner surface 1, the region of outer surface 2, or an intermediate stratum as shown by the position of region b in Figure 10.

In material science, there is a distinction between material porosity and permeability. Porosity is a direct measure of the physical void volume contained with a boundary, whereas permeability refers to the accessibility of that void volume. Permeability is usually expressed as a rate of flow of liquid or gas per unit area, as a function of differential pressure.

In a porous, fibrous material, that part of the total porosity which is available to fluid flow is also called the "effective porosity." The pressure required to force a liquid into a pore is a function of pore size and geometry, liquid surface tension, and solid/liquid contact angle. Surface tension opposes the entry of any nonwetting liquid into a pore, and this opposition may be overcome by external pressure.

Expanded PTFE material is characterized by lengthwise-oriented fibrils interrupted by transverse nodes. The pore size in microns is typically determined by measuring fiber length between the nodes (intemodal distance). To compute fibril length, the material is viewed under sufficient magnification. A fibril length is measured from one edge of one node to the edge of an adjacent node. Fibril lengths are measured from the sample to compute a mean fibril length.

Nodes and fibrils may be further characterized by their relative geometry. That is, nodes by length, width, and height; and fibrils, by diameter and length. It is the relative geometry of nodes to fibrils, as well as, internodal distance that determines porosity and permeability of porous PTFE.

Permeability to fluid flow can be determined by measuring the amount of pressure required for water to permeate the pores of the material. To compute water entry pressure (WEP) one subjects the material to an incrementally increasing water pressure until small beads of water appear on the surface. WEP is a gage which can be used to equate porosity to permeability.

Vascular graft porosity is a measure of the void fraction within the prosthesis wall and is believed to give a rough prediction of the capacity of the graft to anchor newly formed surrounding tissue after implantation, whereas permeability is associated with fluid flow through the graft wall.

Vascular permeability or hydraulic conductivity is related to material porosity. WEP is a good measuring technique to assess this trait because it closely mimics the permeation process at the blood/prosthesis interface. WEP is defined as the pressure value necessary to push water into the pores of a synthetic tubular substrate and can be classified as: High 400 mm Hg), Medium (200-400 mm Hg), and Low (<200 mm Hg).

It has been widely accepted since the nineteenth century that the hydrostatic pressure difference across the arterial wall is capable of transporting water from the blood into the surrounding interstitial space. The view has long been held that a continuous transport of material occurs across the arterial wall, from its inner to its outer surface. Solutes flow past the endothelium gradually passing through the various arterial wall layers eventually being transferred to the lymphatics or adventitia.

The filtration coefficients of the wall are dependent on the hydraulic conductivity of both the intima and media. The artery wall is a heterogeneous porous medium in which interstitial fluid can flow through the interstices between cells and tissue mimicking a semipermeable membrane with hydrostatic and osmotic pressure components. The osmotic pressure difference across the vessel wall is assumed to be small compared with the hydrostatic pressure or hydraulic conductivity.

More controlled healing and tissue ingrowth is achieved by providing a specific region (outer) for cell penetration, followed by a region (barrier) that does not allow free cellular penetration/permeation but instead, allows the transport of plasma solutes such as cellular mediators (proteins, growth factors, etc.) This barrier minimizes the relatively large hydraulic force present in arterial transport that retards tissue ingrowth. Reports have shown that a negative pressure exists within the perigraft space, while blood components (cells, particles, etc.) are isolated to the blood side of the device.

A vascular graft formed from the lamellate structure of the invention mimics the natural artery with a cross-section that offers differential permeability properties resulting in a healing response acceptable to the surrounding tissue.

In a prototype embodiment of the invention, a prosthesis 10 as described above was fabricated in a multi-step procedure by assembling three physically separate bodies of material together in successive strata and then joining or coalescing them into a single unit.

When a vascular prosthesis is fabricated according to this method, preferably at least one of the bodies is a tube which may, for example, be an axially-stretched tube having a porous structure of internodal space oriented transverse to its surface. Advantageously, the nodal spacing, orientation or structure of successive strata may be offset, non-matching or misaligned to introduce or enhance a barrier or hydraulic modulation effect. For example, a prosthesis may be formed by placing a first PTFE tube on a mandrel, wrapping a ribbon of PTFE in an overlapped or non-overlapped spiral winding over the tube outer surface, and then placing another PTFE tube over the assembly. For this construction, the outermost tube has preferably been previously radially expanded. Heat is then applied to the assembly, optionally with a radial compressive force, to shrink back the outer tube and coalesce the three separate bodies together into a unitary prosthesis. Although effectively "welded" together, there are no visible deformations, and the through-wall properties change abruptly at the interface of each stratum or region with the next.

### EXAMPLE 1

PTFE resin (Fluon CD-123 obtained from ICI Americas) was blended with 100 grams of "Isopar H odorless solvent (produced by Exxon Corporation) per pound of PTFE, compressed into a preform billet and extruded into a 3.5 mm I.D. and 4.0 mm O.D. tube in a ram extruder having a reduction ratio of about 200:1 in cross-sectional area from billet to extruded tube. After removal of lubricant, the extruded tube was expanded and sintered, according to the method described in US Patents No. 5,433,909 and 5,474,824, which patents are hereby incorporated by reference herein in their entirety, under various conditions to produce material with different hydraulic porosities. This produced three different tubes, denoted A, B and C, which were used as starting materials for the constructions described below.

Stretch conditions and resultant hydraulic porosities are given below in Table 1.

**TABLE 1**

| | Expansion | | | Hydraulic Porosity |
|---|---|---|---|---|
| | Temp(°C) | Rate(in/sec) | Ratio(%) | WEP |
| | | | | (mm Hg) |
| (A) | 320 | .004 | 3:1 | 100 |
| (B) | 300 | .018 | 3:1 | 200 |
| (C) | 250 | 7.5 | 2.5:1 | 600 |

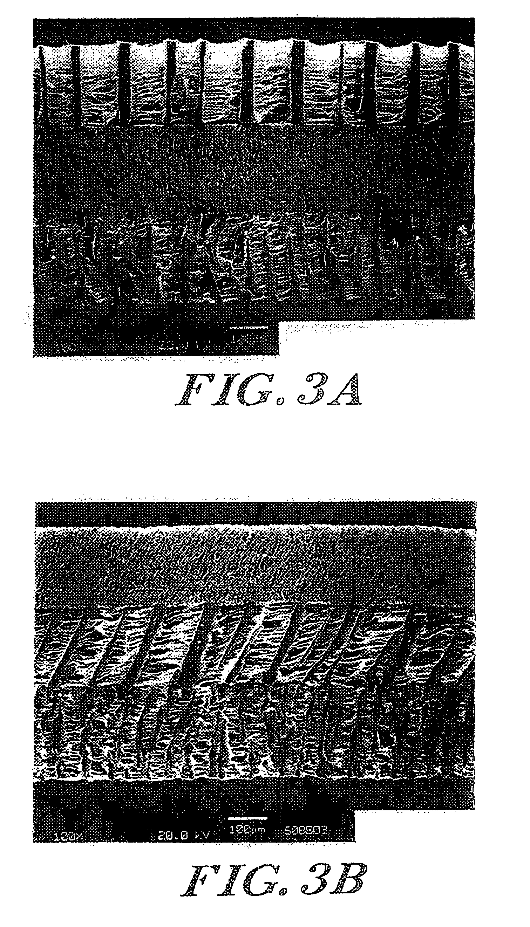

Material (B) was radially expanded to a 4mm ID on a stainless steel forming mandrel, covered with material C that had been previously dilated to a 5mm ID, restrained to prevent longitudinal shrinkage, and transferred to an oven at 360°C for 5 minutes, to prepare a primary lamellate. The primary lamellate was removed from the oven and allowed to cool, covered with material (A) that had been previously dilated to a 5mm ID, restrained to prevent longitudinal shrinkage, and placed in an oven at 360°C for 10 minutes, to prepare a final lamellate structure (material B/C/A), a cross-section of which is shown in Figure 3A.

Material (B) was radially expanded to a 4mm ID on a stainless steel forming mandrel, covered with material (A) that had been previously dilated to a 5mm ID, restrained to prevent longitudinal shrinkage, and transferred to an oven at 360°C for 5 minutes, to prepare a primary lamellate. The primary lamellate was removed from the oven and allowed to cool, covered with material C that had been previously dilated to a 5mm ID, restrained to prevent longitudinal shrinkage, and placed in an oven at 360°C for 10 minutes, to prepare a final lamellate structure (material B/A/C), a cross-section of which is shown in Figure 3B.

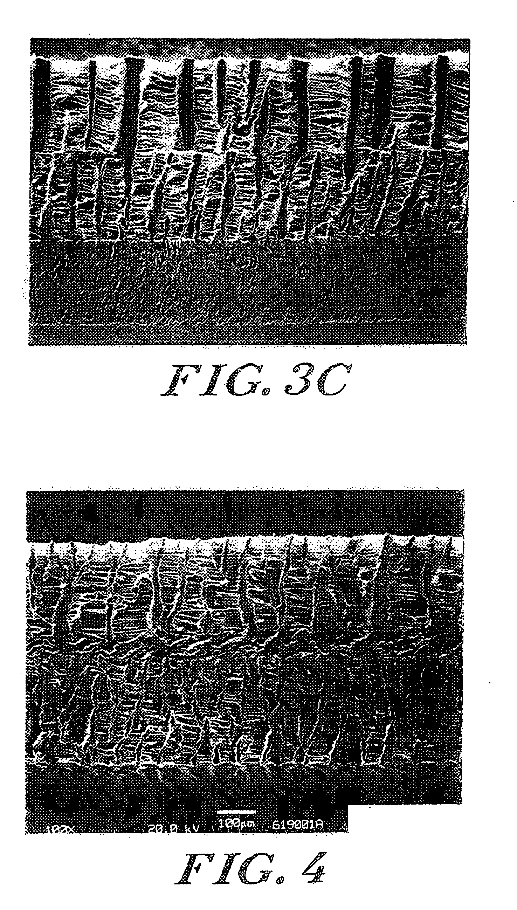

Material (C) was radially expanded to a 4mm ID on a stainless steel forming mandrel, covered with material (B) that had been previously dilated to a 5mm ID, restrained to prevent longitudinal shrinkage, and transferred to an oven at 360°C for 5 minutes, to prepare a primary lamellate. The primary lamellate was removed from the oven and allowed to cool, covered with material (A) that had been previously dilated to a 5mm ID, restrained to prevent longitudinal shrinkage, and placed in an oven at 360°C for 10 minutes, to prepare a final lamellate structure (material C/B/A) a cross-section of which is shown in Figure 3C.

Thus, the three structures of this example differ as permutations of starting materials (A), (B), and (C) assembled into a the tubular prosthetic device, achieving three different articles with differing surface compatibility properties and through permeation profiles to affect tissue growth or biocompatibility..

### EXAMPLE 2

Material (B) was radially expanded to a 4mm ID on a stainless steel forming mandrel, biaxially wound with commercially available PTFE ribbon on a helix winding apparatus, and covered with material (A) that had been previously dilated to a 5mm ID, restrained to prevent longitudinal shrinkage and placed in an oven at 360°C for 10 minutes to prepare a lamellate structure (Material B/Biaxial wrap/Material A), a cross-section of which is shown in Figure 4.

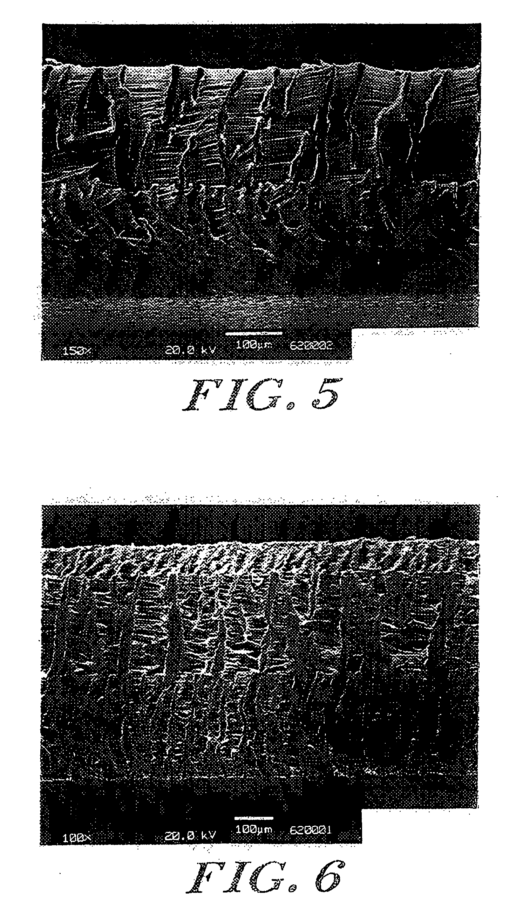

PTFE ribbon was biaxially wound onto a stainless steel forming mandrel, covered with material (B) that had been previously dilated to a 5mm ID, restrained to prevent longitudinal shrinkage and placed in an oven at 360°C for 5 minutes, to prepare a primary lamellate. The primary lamellate was removed from the oven and allowed to cool, covered with material (A) that had been previously dilated to a 5mm ID, restrained to prevent longitudinal shrinkage and placed in an oven at 360°C for 10 minutes to prepare a final lamellate structure (Biaxial ribbon/ material B/material A), a cross-section of which is shown in Figure 5.

Material (B) was radially expanded to a 4mm ID on a stainless steel forming mandrel, covered with material (A) that had been previously dilated to a 5mm ID, restrained to prevent longitudinal shrinkage, and placed in an oven at 360°C for 5 minutes, to prepare a primary lamellate. The primary lamellate was removed from the oven and allowed to cool, covered with a biaxial wrap of PTFE ribbon, restrained to prevent longitudinal shrinkage and placed in an oven at 360°C for 10 minutes to prepare the final lamellate structure (Material B/Material A/Biaxial ribbon), a cross-section of which is shown in Figure 6.

### EXAMPLE 3

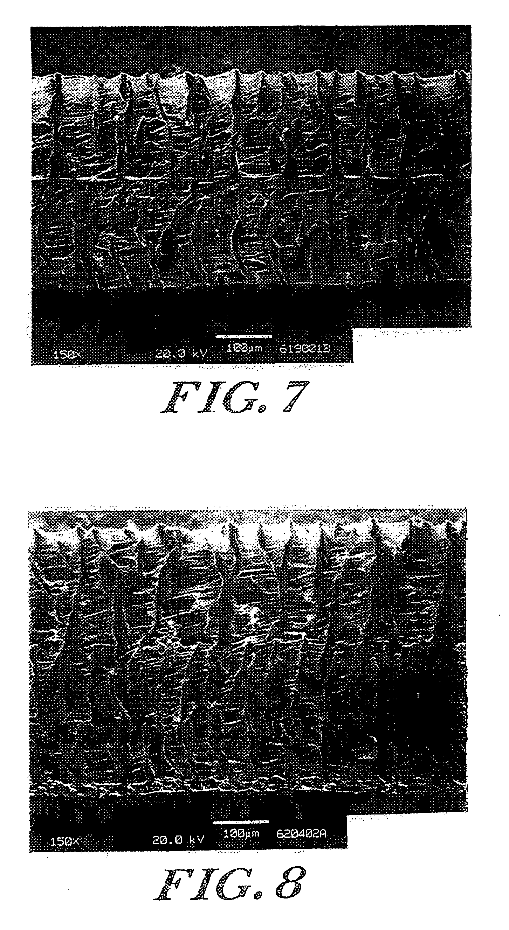

Material (B) was radially expanded to a 4mm ID on a stainless steel forming mandrel, longitudinally wrapped with commercially available PTFE ribbon, and covered with material (A) that had been previously dilated to a 5mm ID, restrained to prevent longitudinal shrinkage, and placed in an oven at 360°C for 10 minutes to prepare a lamellate structure (Material B/Longitudinal wrap/Material A), a cross-section of which is shown in Figure 7.

PTFE ribbon was placed longitudinally around a stainless steel mandrel, covered with material (B) that had been previously dilated to a 5mm ID, restrained to prevent longitudinal shrinkage, and placed in an oven at 360°C for 5 minutes, to prepare a primary lamellate. The primary lamellate was removed from the oven and allowed to cool, covered with material (A) that had been previously dilated to a 5mm ID, restrained to prevent longitudinal shrinkage and placed in an oven at 360°C for 10 minutes to prepare a final lamellate structure (Longitudinal ribbon/Material B/Material A), a cross-section of which is shown in Figure 8.

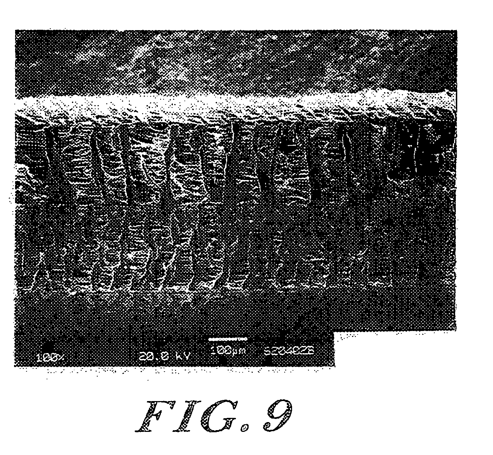

Material (B) was radially expanded to a 4mm ID on a stainless steel forming mandrel, covered with material (A) that had been previously dilated to a 5mm ID, restrained to prevent longitudinal shrinkage, and placed in an oven at 360°C for 5 minutes, to prepare a primary lamellate. The primary lamellate was removed from the oven and allowed to cool, covered with a longitudinal wrap of PTFE ribbon, restrained to prevent longitudinal shrinkage and placed in an oven at 360°C for 10 minutes to prepare a final lamellate structure (Material B/Material A/Longitudinal ribbon), a cross-section of which is shown in Figure 9.

To assess the *in vivo* performance of prostheses prepared in this fashion, four millimeter lamellate grafts of various configurations were implanted into the carotid and/or femoral arteries of dogs. Explants were taken at 14, 30, 60, and 180 days. The presence of an intrawall low porosity, high WEP region produced enhanced tissue ingrowth compared to material without such a region, leading applicant to believe that hydraulic forces play a role in the healing process of implantable devices.

As applied to a covered or integral support prosthesis, the invention provides advantages of strength, expandibility and enhanced tissue compatibility. As shown in Figures 11A-11C, a method of forming an enclosed, protected support or reinforcing element 11 such as a stent includes the steps of (Figure 11A) taking a first film-like body of liner material shown as a tube 20a, and placing it within a stent 35, and the step (Figure 11B) of covering the assembly with an outer film of material, 20b. The entire assemblage is then heated together (Figure 11 C) so as to unitize the inside and outside layers with the stent 35 secured between them. The foregoing schematically represented method is preferably carried out with a material such as expanded polytetrafluoroethylene (PTFE) which has a porosity imparted by previous stretching or expansion of the material. In this case, at least the second or outer layer of the material is preferably a radially-expanded but unsintered layer, so that when it is heated it shrinks back toward its unexpanded state and presses against the inner layer. Further radial pressure may be provided to urge the inner and outer polymer layers together.

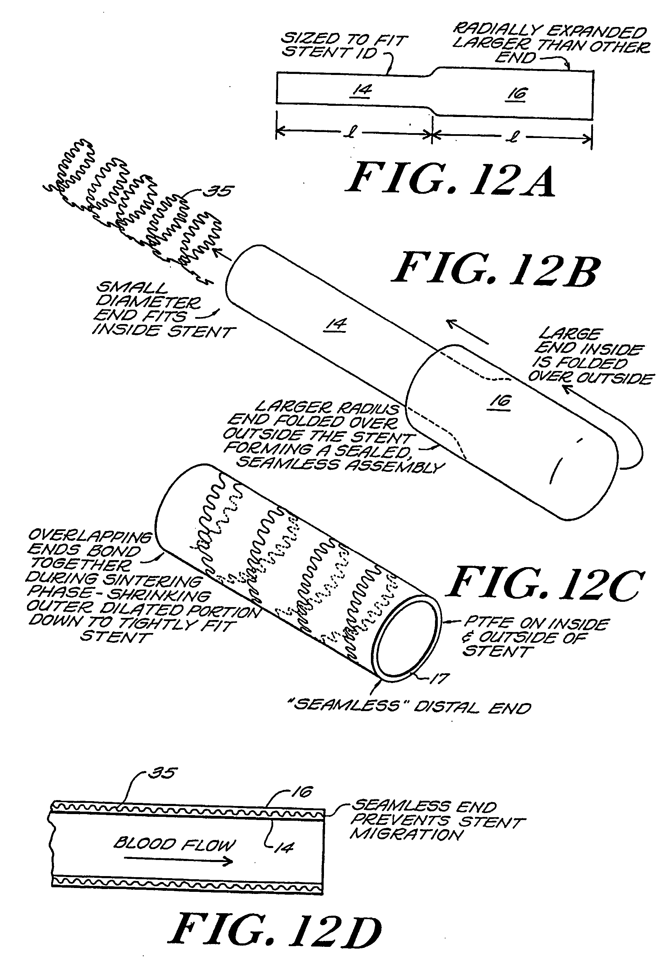

One preferred method of fabricating the structure schematically shown in Figures 11A-11C is illustrated in Figures 12A-12D. In accordance with this method, a tube of the polymer material having a diameter d is provided. One end of the tube is expanded radially, for example by inserting an expandable balloon inside the tube and inflating it to increase the diameter of the tube by a factor of five to five hundred percent or more. This produces a stepped tube illustrated in Figure 12A having a small diameter portion 14 of diameter d and a large diameter end 16 of diameter d, which may in addition have a greater degree of porosity. A stent 35 is then placed over the small end 14 and the large end 16 is folded back over the stent 35 (Figure 12B). This forms a structure that is half the original tube length, with a single cuff resulting from the continuous fold of material 16 over the right hand end portion, as illustrated, of the device. Once folded over in this fashion to form an assembly half the length of the original tube, heat is applied to shrink the outside down in upon the inside, enclosing the stent 35 therebetween (Figure 12C). An inflatable sleeve or tightly fitting form may clamp around the outside to provide an inwardly-directed, radial, pressure. Figure 12D illustrates a cross section taken longitudinally of the resultant construction.

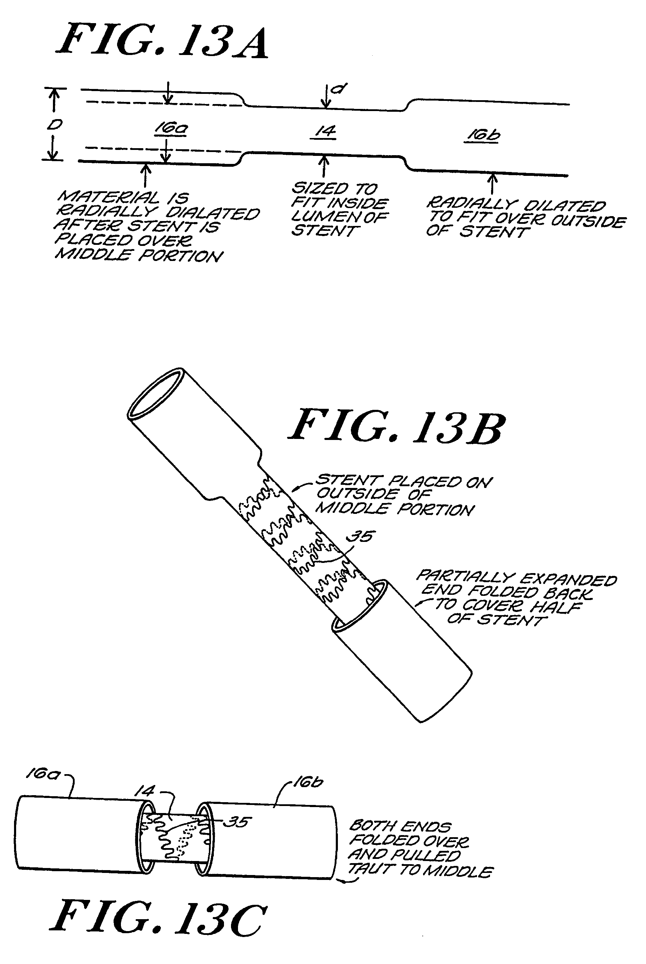

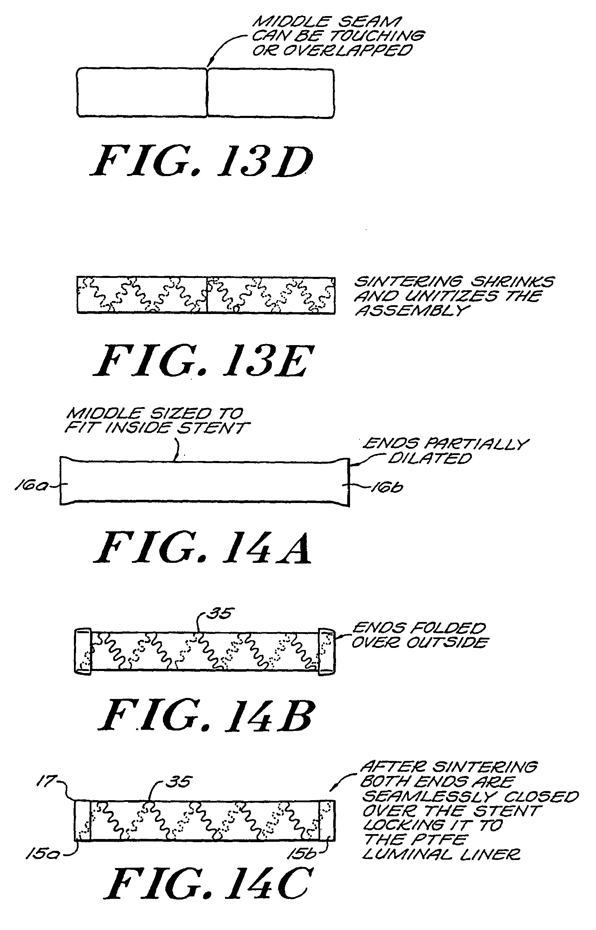

Figures 13A-13E show a further practice of the method of the present invention. In accordance with this method, a single tube of polymer material is provided as before, but both ends are inflated to form a first large diameter portion 16a that joins continuously with the uninflated central portion 14, which, in turn, extends to another end 16b which has also been inflated and enlarged. Preferably, the portion 14 extends for approximately half the length of the original tube while the portions 16a and 16b are each one quarter of the length of the tube length or slightly more. This tube may be placed over a mandrel (not shown) which provides a temporary rigid element to facilitate the process. As shown in Figure 13B, the stent 35 is then placed around the central portion 14 and one end, illustrated as end 16b of the expanded tube, is folded back along the axial direction to cover a portion of the stent. As illustrated, the folded back end portion 16b extends roughly halfway along the tube length. As best seen in Figures 13C and 13D, the remaining end 16a is then folded and pulled taut to the middle. The ends can be touching as shown in Figure 13D, or they may be overlapped to provide a single seam in which one end slightly extends over the already double layer of the other end. The assembly then remains on the mandrel and is heated for a time sufficient to shrink both of the turned-over ends together down over the stent and to coalesce with the underlying material. With this construction, both ends of the stent are closed by a continuous smooth seamless cover, and both the inside and outside films are bonded to each other or coalesced with heat so that they form a nondelaminating and unitized cocoon around the stent.

In this construction, the stent body 35 itself may be a spiral-shaped zig-zag wire body which lies generally in the plane of the cylindrical surface on which it extends, and which when radially expanded places the bends under tension and draws the band of zig-zags slightly narrower and straighter, thus expanding in radius by elongating slightly along its spiral direction. Shear between the surrounding polymer layers and the wire stent material itself will thus naturally occur, but will be directed along the relatively narrow band in which the stent lies. The crimps themselves may be of very closely spaced zigs and zags which effectively prevent the outer film from contacting the inner film in the narrow band closely surrounding this area. With such a construction, the stent lies in a tunnel or pocket formed between the layers. Because the layers fit tightly, the support effectively transfers strain to the polymer. Thus breaking or rupture of the film does not occur as the stent expands. As noted above, a preferred material is an expanded polytetrafluoroethylene, which when heated shrinks back and coalesces with contacting portions of the polymer from the other side of the stent. The heating is carried out to not over sinter, so this material is also capable of restretching without rupture. Thus that both the stent and the surrounding polymer are expandable and may, for example, be placed by endovascular delivery and expansion *in situ.*

In addition to the foregoing methods of fabrication, the invention also contemplates a stent construction wherein the stent body has a continuous and seamless covering over ends of the stent and along the full length of the body, but the body covering extends only on one side, the inside or the outside, of the cylindrical stent. This is achieved as shown in Figures 14A-14C. In this embodiment of the invention, a tube 14 is expanded at each end, as before, to form expanded end portions 16a and 16b. However the end portions, 16a and 16b, are each of relatively short length, approximately one centimeter, and are folded back over the stent 35 only for a distance sufficient to cuff the ends and to provide a short band or margin approximately one half to one centimeter wide at the ends of the stent. As before, the assembly is then heated to shrink down the folded over material and unitize the cuffs thus formed at each end. In this embodiment as in the first described schematic treatment of the method, a tube may then be placed over the outside to cover the folded back portions. If such an outer tube is provided, it is constrained so that its ends extend beyond the edges of the folded back cuff portions and lie in the band indicated by 15a, 15b in the drawing. Heat is applied with the ends constrained so that the second tube shrinks radially but not axially and the cocoon structure extends and is maintained over the full length of the prosthesis. As in the previous embodiments, the closed ends are entirely seamless and the outer tube coalesces with the underlying material at a region away from the ends to form a continuous enclosing assembly for the stent.

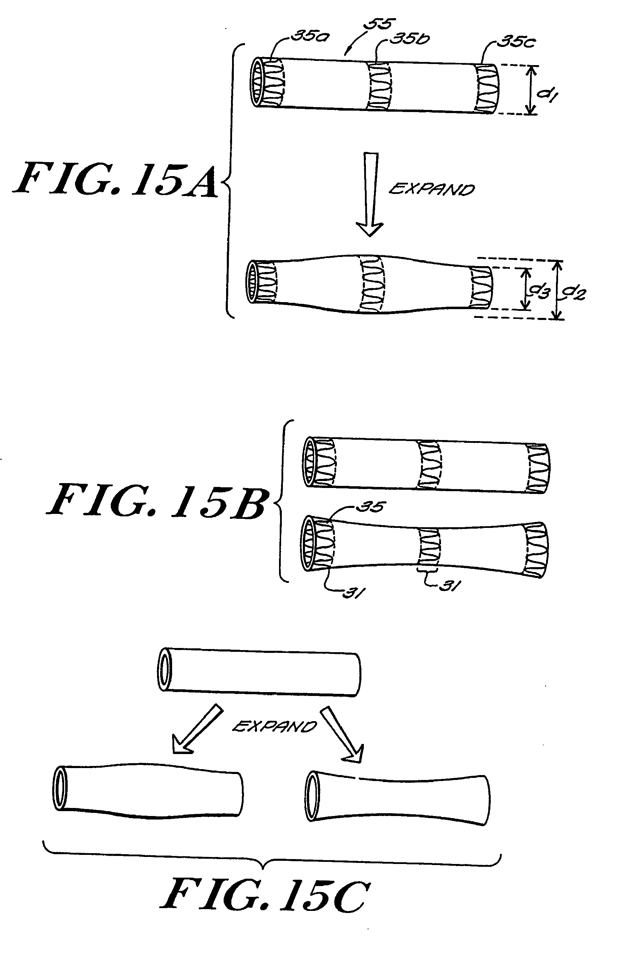

In accordance with another aspect of the present invention, a vessel support is fabricated with a structure to assume a radially-varying extent along its length when expanded, such that one or both ends thereof are larger than its center as shown in Figure15A. A corresponding construction may be used to form a support which upon expansion assumes the opposite flare or bulge, as shown in Figure 15B.

As illustrated in Figure15A, a PTFE support liner or graft assembly 55 is fabricated having one or more expanding rings or stents 35a, 35c at its ends and one or more rings 35b centrally along its length, all having an initial diameter d₁. In this embodiment the end rings 35a, 35c are made of heavier gauge material, or are otherwise dimensioned so that they either are more resistant to expansion, or else have a limited net expanded size, d₂. The central rings 35b are either more easily expanded (lower resistance) or are dimensioned so that they expand to a larger diameter d₃. The result is that during expansion the support assembly 55 assumes a shape which bulges out in the center. This shape aids in preventing the inflation balloon from slipping out of the support during the inflation process, which may require several cycles of balloon expansion to expand successive lengths of the support tube. This contour also enables the support to conform more closely to a region of vessel having an aneurysm or bulge.

Figure 15B illustrates a related construction in which the end rings have a lesser resistance to expansion or a larger net expanded diameter than the center rings. In this case the intermediate and/or final expanded size is greater at the ends, creating flared or trumpet-shaped ends or a venturi-like profile. This profile is intended to assure a smooth transition from the unsupported vessel lumen to the prosthesis, without projecting edges at the prosthesis ends. Each of the narrow rings 35 may reside in a pocket or band 31 such that expansion occurs relatively freely and some motion of the stent may occur between the inner and outer surfaces without impairing continuity of the PTFE envelope material.

A related construction, applicable to either bulge- or flare-expansion contours is shown in Figure 15C. This Figure shows a PTFE body which has been formed with an inner region, a middle region and an outer region, as described for example above with refence to Figures 3-10. That construction, instead of enclosing stent or rings 35, surrounds a separate tube, wrapping or body of fluoropolymer material, in the region between the outer and inner surface portions in a unitary coalesced assembly. In this case, when constructing the assembly as shown in Figure 15C without a stiff stent or support member between the inner and outer surfaces, the degree of expansion of the assembly may be varied by providing the prosthesis with a different degree of sintering at different points along the length of the prosthesis. This may be done as described further below, by feeding the assembly end-first into a sintering oven at a controlled rate to sinter each end more than the center. This provides greater tensile strength of the material at both ends, and results in a bulged prosthesis when expanded, as shown at the left of the Figure. The opposite distribution may be provided by reciprocating the assembly while it is centrally positioned in a short oven such that each end sticks out of the heat zone for a substantial portion of the sintering cycle, to result in flared-end expansion material at the end portions having lower tensile strength.

Thus, the invention further provides expandable vessel liners or supports having radial taper or curvature along their length.

### Example A

The following example describes the construction of a solid tube of PTFE having a radially expandable stent within the tube. A starting tube, or substrate, was a substantially uniform length of axially-stretched tubing formed by extrusion as described, for example, in U.S. Patents No. 5,433,909 and 5,474,824. The PTFE substrate was a uniaxially-oriented tube having nodes interconnected by fibrils oriented substantially in a single longitudinal direction. The fibrils were approximately 30µm in length. The PTFE substrate had an internal diameter of 2.5 mm and a wall thickness of approximately 0.25 mm.

This PTFE substrate material was placed on a stainless steel mandrel having a diameter of 2.7 mm. Thus, a small amount of radial expansion was required to get the material on the mandrel. A Cordis peripheral stent (part number 2969363) was placed over PTFE layer, and a second PTFE layer was placed covering the stent. The second layer consisted of the PTFE substrate material described above, expanded to 4 mm by sliding the material over a stainless steel mandrel with a diameter of 4 mm to radially expand it. After radial expansion, the PTFE material moved freely over the stent. The PTFE/stent/PTFE combination was secured at both ends to prevent slippage along the mandrel, and the combination was placed in an oven and sintered at 360° C for a 15 minute time period. During sintering, the outer layer of PTFE recovered to its original diameter and attached to the inner layer of PTFE. The assembly was removed from the oven and the excess PTFE was removed. The resulting structure contained the stent entirely enveloped within a closed body of PTFE.

The PTFE stent combination was expanded with a Blue Max balloon dilatation catheter with a balloon O.D. of 9 mm and a balloon length of 4 cm. Using multiple inflations the stent combination was expanded along its entire length.

### Example B

The following example describes the construction of a solid tube of PTFE having a radially expandable stent within the tube, and wherein a single piece of PTFE tubing is used to construct the stent combination.

The PTFE substrate was the same uniaxially-oriented tube as that of Example A, having nodes interconnected by fibrils oriented substantially in a single longitudinal direction. The fibrils were approximately 30 µm in length, and the PTFE substrate had an internal diameter of 2.5 mm and a wall thickness of approximately 0.25 mm. Half the length of the PTFE tube was expanded to 6 mm by sliding the tube material over a mandrel having a diameter of 6 mm. Figure 12A depicts what the PTFE tube looks like after this processing step. The small portion of the PTFE tube was then placed on a stainless steel mandrel having a diameter of 2.7 mm. Thus, a small amount of radial expansion was required to get the material on the mandrel. A Cordis peripheral stent (part number 2969363) was placed over the PTFE layer. Next, the expanded end of the PTFE tube was folded back to cover the stent. The PTFE material moved freely over the stent, and the PTFE and stent combination was secured at both ends to prevent slippage along the mandrel. The combination was placed in an oven and sintered at 360° C for a 15 minute time period. During sintering, the outer layer of the PTFE recovered to its original diameter and attached to the inner layer of PTFE.

The PTFE and stent combination was removed from the oven and excess PTFE was trimmed.

The PTFE stent device produced in this fashion was expanded with a Blue Max balloon dilatation catheter with a balloon O.D. of 9 mm and a balloon length of 4 cm. Multiple inflations were used to expand the stent combination along its entire length.

### Example C

The following example describes the construction of a solid tube of PTFE having a plurality of spaced apart radially expandable stents within the tube.

The starting PTFE substrate was an uniaxially-oriented tube having nodes interconnected by fibrils oriented substantially in a single longitudinal direction. The fibrils were approximately 30 µm in length. The PTFE substrate had an internal diameter of 2.5 mm and a wall thickness of approximately 0.25 mm.

A layer of this PTFE material was placed on a stainless steel mandrel having a diameter of 2.7 mm. Thus, a small amount of radial expansion was required to get the material on the mandrel. Two Palmaz stents (part number P394) were placed over the PTFE layer. The stents were spaced apart approximately 4 cm. The PTFE material described above was expanded to 4 mm by sliding the material over a stainless steel mandrel with a diameter of 4 mm and the expanded tube was placed over the stents. The radially expanded PTFE material moved freely over the stents. The PTFE and stent combination was secured at both ends to prevent slippage along the mandrel. The combination was placed in an oven and sintered at 360° C for a 15 minute time period. During sintering, the outer layer of PTFE recovered to its original diameter and attached to the inner layer of PTFE. The PTFE and stent combination was removed from the oven and excess PTFE was removed.

The PTFE stent combination was expanded with a Blue Max balloon dilatation catheter with a balloon O.D. of 9 mm and a balloon length of 4 cm. Multiple inflations were used to expand the stent combination along its entire length.

### Example D

The starting PTFE substrate was made from a uniaxially-oriented tube having nodes interconnected by fibrils oriented substantially in a single longitudinal direction. The PTFE substrate was 60 cm in length had an internal diameter of 6 mm, with a wall thickness of approximately 0.6 mm and an average internodal distance of 60 µm on the exterior surface and 20µm on the interior surface. The tube material was marked 1 cm increments along the length of the tube.

This graft material was placed on a stainless steel mandrel having a diameter of 6 mm which allowed for the free movement of the graft along the length of the mandrel. The graft/mandrel combination was then fed along its long axis into and oven set at 360° C at a rate of 0.1 cm/sec. Consequently, the portion of the tube entering the oven initially is subjected to a longer sintering time than other portions of the tube. The tube was not restrained during sintering which permitted longitudinal contraction, which is also referred to as free sintering. Free sintering results in a reduction in the internodal distance.

Samples of the tube were taken at points 12.5, 22.5 and 52.5 centimeters along the longitudinal axis. Thus, the samples had been subjected to different free sintering times. Scanning electron micrographs (SEMs) were made to observe the effect of sintering on the PTFE material.

### Example E

An expanded PTFE tube was made with variable porosity characteristics. The PTFE substrate was made from a uniaxially-oriented tube having nodes interconnected by fibrils oriented substantially in a single longitudinal direction. The PTFE substrate was 100 cm in length had an internal diameter of 6 mm, a wall thickness of approximately 0.45 mm and an average internodal distance of 60 µm on the exterior surface and 20 µm on the interior surface. The tube was marked in 0.5 mm increments along the length of the tube.

The graft was placed on a stainless steel mandrel having a diameter of 6 mm which allowed for the free movement of the graft along the length of the mandrel. The graft mandrel combination was then fed along its long axis into an oven set of 320° C at a rate of 0.05 cm/sec to the 50 cm position. Consequently, the portion of the tube entering the oven initially was subjected to a longer sintering time than other portions of the tube. The tube was not restrained during sintering, which permitted longitudinal contraction. This is referred to as free sintering. Free sintering results in a reduction in the internodal distance. The tube mandrel combination was rotated 180°. The graft mandrel combination was then fed along its long axis into an oven set at 320° C at a rate of 0.05 cm/sec to the 50 cm position. This permitted the free sintering of the opposite section of tube material in a similar fashion to the initial section. Thus, the center portion of the tube was subjected to the shortest period of free sintering.

Samples of the tube material were taken at various positions along the longitudinal axis and subjected to radial tensile strength, foreshortening, and internodal distance measurements, and the radial tensile strength was found to be directly related to the foreshortening percentage and internodal distance.

As indicated in the above examples, the densities and porosities of the PTFE zones maybe varied to meet the specific needs of a particular prosthesis. The foregoing constructions and methods provide new and useful constructions for reinforced prostheses, prostheses having wall pore structures that vary in diverse physiologically important physical characteristics, and for stents and other solid or springy supports with a cover or surrounding of PTFE material. This surrounding material is permanently attached and closely conforms, providing a surface biocompatibility and a continuous surface that is capable of expansion while retaining its integrity, and which essentially avoids defects and surface seams or irregularities. Moreover, the inner portion may have a high WEP and operate to inhibit diffusion or modulate biological response and growth processes occurring in or contiguous to the wall. As such, it provides an improved construction applicable to a broad range of implant and surgical protheses. The invention being thus disclosed and described, variations and modifications will occur to those skilled in the art, and such variations and modifications are considered to be within the scope of the invention, as set forth in the claims appended hereto.

Preferred embodiments and preferred aspects of the invention:
1. An improved tubular prosthesis for surgical implantation to replace a segment of a blood vessel, such prosthesis comprising
   a first member constituting a flow passage extending along a tube axis and formed of porous polymer
   at least one support bead disposed on an exterior surface of said first member and extending circumferentially at a regular pitch to formspaced apart transverse ridges having a profile protruding above said exterior surface of said first member, and
   a polymer membrane placed over said support bead and coalesced to said tube said polymer membrane adhering over the ridges and bonding to said first member to enclose said ridges and provide kink and compression resistance without interlaminar peeling, said membrane further modulating said profile to a smooth undulation for atraumatic tunneling and enhanced surgical handling to reduce abrading of surrounding tissue.
2. A tubular prosthesis according to aspect 1, wherein said support bead includes a metal wire, which is optionally polymer coated and under tension.
3. A tubular prosthesis according to aspect 1, wherein said support bead is a bead of solid, non-porous and unexpanded PTFE.
4. A tubular prosthesis according to aspect 2 or 3, wherein said support bead has a diameter between .025 and 1.0 millimeter.
5. A tubular prosthesis according to any of aspects 1-5, wherein said support bead is wound with a pitch effective to trap a needle from sliding axially and thereby to prevent tearing along the axis of the tube when the prosthesis is subject to cannulization.
6. A tubular prosthesis according to aspect 1, wherein said first tubular member, said support bead and said membrane are coalesced by heat without additional solvent to substantially unitize the prosthesis against delamination.
7. A tubular prosthesis according to aspect 1, wherein said prosthesis has a diameter between one half and forty millimeters.
8. A tubular prosthesis according to aspect 7, wherein said polymer membrane has thickness under 1.0 millimeters.
9. A tubular prosthesis according to aspect 7, wherein said polymer membrane has thickness under two millimeters.
10. A tubular prosthesis according to aspect 7, wherein said first tubular member has a thickness under 0.2 millimeters.
11. A tubular prosthesis for surgical implantation to replace a segment of a blood vessel, such prosthesis comprising
   a first tubular member constituting a flow passage extending along a tube axis and formed of porous polymer
   at least one body of fluoropolymer extending circumferentially about and forming spaced apart transverse ridges disposed on and protruding above an exterior surface of said first tubular member, and
   a polymer membrane placed over said body and coalesced to said tubular member to provide a continuous undulant cover adhering over the ridges to modulate the external profile of the assembled prosthesis for atraumatic tunneling , and said ridges being dimensioned and positioned with a pitch effective to direct a dialysis needle to a puncture site without substantial ploughing, hole enlargement and shape deformation, whereby the tubular prosthesis heals to surrounding tissue and provides inproved dialysis needle access while avoiding tunnel hematomas.
12. A tubular prosthesis according to aspect 11, wherein said fluoropolymer material has an elastic limit of deformation greater than that of said polymer.
13. A tubular prosthesis for surgical implantation to replace a segment of a blood vessel, such prosthesis comprising
   a first tubular member constituting a flow passage extending along a tube axis and formed of porous polytetrafluoroethylene (PTFE)
   at least one bead of fluoropolymer extending circumferentially about and forming transverse ridges disposed on and protruding above an exterior surface of said first tubular member at a regular pitch, and
   a PTFE membrane placed over said bead and coalesced to said first tubular member to provide a continuous undulant cover adhering over the ridges that modulates external profile of the assembled prosthesis to a reduced friction smooth surface profile while enhancing tissue ingrowth at said external profile.
14. A method of forming a tubular prosthesis, such method comprising the steps of
   providing a polymer tube,
   placing the polymer tube around a mechanical support,
   winding at least one bead of fluorpolymer material on an exterior surface of said tube in a helical or circumferential pattern of discrete, axially spaced-apart, protruding ridges on said exterior surface,
   placing an outer polymer tube over said ridges to enclose and form a continuous sheath thereover, and
   coalescing said polymer tube and said outer polymer tube thereby enclosing and smoothly covering said ridges therebetween.
15. The method of aspect 14, wherein the step of coalescing includes heating the inner tube, the bead and the outer tube on said mechanical support to shrink the outer tube tightly about the bead.
16. The method of aspect 14, wherein the step of winding includes placing discrete rings at an axial spacing between one and five millimeters to form a segmented supporting structure.
17. The method of aspect 14, wherein said bead of fluoropolymer material includes a metal wire core enveloped by a PTFE coating.
18. A method in accordance with aspect 14, wherein the step of winding includes winding said bead with a pitch between approximately one and three millimeters effective for the ridges to trap a needle and prevent longitudinal tearing of the prosthesis when cannulized with a dialysis needle.
19. A method of forming a tubular prosthesis, such method comprising the steps of
   providing a PTFE tube
   placing the PTFE tube around a support
   winding at least one bead of fluoropolymer material onto an exterior surface of said tube in a helical or circumferential pattern of discrete, axially spaced-apart, protruding ridges on said exterior surface
   placing an outer PTFE tube over said ridges to enclose and form a continuous sheath thereover, and
   coalescing said PTFE tube and said outer PTFE tube thereby enclosing and smoothly covering said ridges therebetween,
   wherein the step of winding includes winding said bead with a pitch between approximately one and three millimeters, effective for the ridges to trap a needle and prevent longitudinal tearing of the prosthesis when cannulized with a dialysis needle.
20. The method of aspect 19, further wherein the step of winding includes winding said bead so that said ridges are effective to direct a needle to a puncture site at an angle which prevents substantial plowing, hole enlarging and shape deformation.
21. A method of forming a tubular prosthesis, such method comprising the steps of
   providing a PTFE tube,
   placing the PTFE tube around a support,
   winding at least one bead of fluorpolymer material on an exterior surface of said tube in a helical or circumferential pattern of discrete, axially spaced-apart, protruding ridges on said exterior surface,
   placing an outer PTFE tube over said ridges to enclose and form a continuous sheath thereover, and
   coalescing said PTFE tube and said outer PTFE tube thereby enclosing and smoothly covering said ridges therebetween.
22. An improved tubular prosthesis for surgical implantation to replace a segment of a blood vessel, such prosthesis comprising
   a first member constituting a flow passage extending along a tube axis and formed of porous polymer
   at least one support bead disposed on an exterior surface of said first member and extending circumferentially at a regular pitch to form spaced apart transverse ridges having a profile protruding above said exterior surface of said first member, and
   a tissue ingrowth membrane placed over said support bead and coalesced to said tube said tissue ingrowth membrane adhering over the ridges and bonding to said first member to enclose said ridges and provide kink and compression resistance without interlaminar peeling, said membrane further modulating said profile for atraumatic tunneling and also promoting fibroblast ingrowth thereby enhancing dialysis needle access of said prosthesis.
23. A prosthetic member comprising
   a prosthesis wall having at least first, second and third portions along a wall thickness,
   said first portion including a porous inner portion extending continuously along an axis
   said second portion being contiguous to said first portion and forming a barrier impenetrable to cell growth
   said third portion lying over said second portion and extending as a continuous body along said axis
   said first, second and third portions being coalesced together to form said wall having cellularly penetrable first and third portions and said second portion modulates cellular penetration of said first and third portions.
24. A prosthetic member according to aspect 23, wherein said second portion barrier has a porosity less than fifty micrometers effective to block growth of cells and block transport of large molecules while allowing gases and small soluble molecules to pass through said second portion.
25. A prosthetic member according to aspect 23, wherein said first and third portions are continuous tubes and said second portion is a biasedwrap.
26. A prosthetic member according to aspect 23, wherein said first and third portions are continuous tubes and said second portion is a non-biased wrap.
27. A prosthetic member according to aspect 23, wherein said first, second and third portions are each continuous tubes.
28. A prosthetic member according to aspect 27, wherein said first and third portions are stretched in at least one direction, and said second portion is substantially unstretched.
29. A prosthetic member according to aspect 27, wherein said first and third portions are stretched in a common direction, and said second portion is stretched to form fibrils which are oriented in said second portion across said common direction.
30. A prosthetic member according to aspect 23, wherein said second portion barrier has a water entry pressure which prevents weeping after said wall has been wetted.
31. A prosthetic member according to aspect 23, wherein said third portion is formed of a porous fluoropolymer having a node and fibril microstructure, wherein the fibrils extend along the axis of said tube and the nodes are oriented to form channels between adjacent nodes, the channels being transverse to said wall and tapering smaller as they extend inwardly.
32. A method of making a device, such method comprising the steps of placing at least first and second porous PTFE portions one over the other with a low porosity PTFE therebetween to form an adhesive-free assemblage , and heating the assemblage to coalesce the assemblage into a unitized device.
33. The method of aspect 32, wherein the device is a prosthesis and said first and second portions are tubes.
34. The method of aspect 32, wherein said substantially non-porous PTFE is a PTFE tube, and the step of placing to form an assemblage includes the steps of placing said tubes on a mandrel, and wherein the step of heating includes heating the assemblage while it is on the mandrel so that force of shrinkage of the tubes against the mandrel when heated joins the tubes together without solvent.
35. The method of aspect 32, wherein the low porosity PTFE has a bias orientation and a thickness below approximately one millimeter.
36. A method of forming a device, such method comprising the steps of providing a first portionof porous PTFE
   providing a second portion of low or non-porous material surrounding said first portion
   providing a third portion of porous PTFE surrounding said second portion, and
   coalescing said first portion, said second portion and said third portion into a single wall wherein said second portion modulates cell growth in portions of said wall formed by said first and thirdPTFE portions and limits through penetration of cell growth.
37. A prosthetic member comprising
   a prosthesis wall formed entirely of fluoropolymer having at least first, second and third portions along a wall thickness,
   said first portion including a porous inner section extending continuously along an axis
   said second portion being contiguous to said first portion and forming a stratum for modulating hydrostatic pressure
   said third portion overlying said second portion and including a porous section extending as a continuous body along said axis
   said first, second and third portions being coalesced together to form said wall such that the wall has a cellularly penetrable surface and said second portion modulates fluid communication through the wall while preventing cellular growth in said second portion.
38. An endostent for insertion into a biological flow passage for expansion to maintain the passage open, such endostent including a radially expandable member shaped and adapted for insertion within the passage and for being expanded radially within said passage to apply outward pressure against walls of the passage when expanded, and a polymer sheath forming a seamless and continuous cuff over an end of said member and tightly enclosing the expandable member between substantially continuous inside and outside layers of said sheath.
39. An endostent according to aspect 38, wherein said sheath comprises an inner lamina of PTFE and an outer lamina of PTFE. The inner lamina and the outer lamina contacting and being bonded to each other at plural distributed contact points thereby forming an integral and non-delaminating assembly with said expandable member.
40. An endostent according to aspect 39, wherein the expandable member comprises a plurality of spaced apart expansion rings, said inner lamina being bonded to said outer lamina only in regions extending between successive ones of said plurality of expansion rings, said rings thereby being free to expand in pockets between said bonded regions.
41. An endostent according to aspect 40, wherein said expansion rings are zig-zag expansion rings which each occupy a band of axial extent when radially expanded, and wherein said inner lamina is spaced apart from and not bonded to said outer lamina throughout each said band whereby the expansion rings are free to expand without shearing said lamina.
42. An endostent according to aspect 38, wherein the expandable member comprises a balloon-expandable tube formed of metal expansion mesh material.
43. An endostent according to aspect 39, wherein said distributed contact points constitute greater than one half of the surface area of said inner lamina.
44. An endostent according to aspect 39, wherein said endostent has a tube shape and said inner lamina loops back over itself to form said cuff and said outer lamina thereby closing an end of the sheath to maintain said member enveloped therein.
45. A method of forming a stent assembly for stenting an anatomical passage, such method comprising the steps of
   providing a radially expandable stent member, and
   enveloping the stent member by
   providing an inner sheet of radially-expandable material
   and
   folding said sheet back to enclose the stent within a cuff,
   the step of enveloping including joining the inner sheet to the outer sheet over a distributed region so that when the stent member is expanded the sheets do not delaminate.
46. An improved stent assembly for stenting an anatomical passage, such assembly including a perforate expansile stent body having a plurality of through apertures, an expandable tube over said body and an inner tube within said body, the inner tube extending seamlessly and continuously over at least one end of the body, and both tubes being joined to each other through said apertures.
47. A method of fabricating a stent, comprising;
   inserting a tube centrally in a stent body
   folding back a portion of the tube over itself so the tube extends inside and outside the stent body forming a cuff seamlessly and continuously enclosing at least one end thereof within a cocoon, and
   coalescing the tube to seal the stent body within the cocoon.
48. The method of aspect 47, wherein the step of folding back a portion includes a step of radially expanding the portion before folding back.
48 The method of aspect 47, wherein the step of coalescing includes a step of heating to contract and join the tube to the stent body.
49. The method of aspect 47, including the step of increasing porosity of said portion of the tube.

## Claims

1. An implantable prosthetic device, comprising:
a wall forming a tubular structure and having at least first, second, and third portions along a wall thickness, the wall having an interior side and an exterior side;
wherein the first portion extends axially along and forms the interior side of the wall, the first portion having a first porosity;
wherein the second portion extends along the first portion and forms a physical barrier impenetrable to cell growth;
wherein the third portion extends along the second portion and has a second porosity, the second porosity being less porous than the first porosity;
the first, second, and third portions being coalesced together to form the wall.

2. The device of claim 1, wherein the wall forming the tubular structure has a diameter between 0.5 mm and 40 mm.

3. The device of claim 1, wherein the wall has cellularly penetrable first and third portions and the second portion modulates cellular penetration of the first and third portions.

4. The device of claim 1, wherein the second portion has a porosity less than fifty micrometers, which is effective to block growth of cells and block transport of large molecules while allowing gases and small soluble molecules to pass therethrough.

5. The device of claim 1, wherein the first and third portions are continuous tubes and the second portion is a biased wrap.

6. The device of claim 1, wherein the first and third portions are continuous tubes and the second portion is a non-biased wrap.

7. The device of claim 1, wherein the first, second, and third portions are each continuous tubes.

8. The device of claim 1, wherein the first and third portions are stretched in at least one direction, and the second portion is substantially unstretched.

9. The device of claim 1, wherein the first and third portions are stretched in a common direction, and the second portion is stretched to form fibrils which are oriented in the second portion across the common direction.

10. The device of claim 1, wherein the second portion barrier has a water entry pressure that prevents weeping after the wall has been wetted.

11. The device of claim 1, wherein the third portion is formed of a porous fluoropolymer having a node and fibril microstructure, wherein the fibrils extend along the axis of the tubular structure and the nodes are oriented to form channels between adjacent nodes, the channels being transverse to the wall and tapering smaller as they extend inwardly.

12. The device of claim 1, further comprising a radially expandable member disposed in such a way that the radially expandable member is enclosed within the wall.

13. An implantable prosthetic device, comprising:
a tubular structure formed by a wall made of fluoropolymer material and having at least first, second, and third portions along a wall thickness;
wherein the first portion extends axially along and forms the interior side of the wall, the first portion having a first porosity;
wherein the second portion extends along the first portion and forms a physical barrier impenetrable to cell growth;
wherein the third portion extends along the second portion and has a second porosity, the second porosity being less porous than the first porosity;
the first, second, and third portions being coalesced together to form the wall in such a way that the wall has a cellularly penetrable surface and the second portion modulates fluid communication through the wall while preventing cellular growth in the second portion.

14. An implantable prosthetic device in the form of an endostent for insertion into a biological flow passage for expansion to maintain the passage open, comprising:
a radially expandable member shaped and adapted for insertion within the passage and for being expanded radially within the passage to apply outward pressure against walls of the passage when expanded; and
a polymer sheath forming a seamless and continuous cuff over an end of the member and tightly enclosing the expandable member between substantially continuous inside and outside layers of the sheath.

15. The device of claim 14, wherein the sheath comprises an inner lamina of PTFE and an outer lamina of PTFE, the inner lamina and the outer lamina contacting and being bonded to each other at plural distributed contact points thereby forming an integral and non-delaminating assembly with the expandable member.

16. The device of claim 15, wherein the expandable member comprises a plurality of spaced apart expansion rings, the inner lamina being bonded to the outer lamina only in regions extending between successive ones of the plurality of expansion rings, the rings thereby being free to expand in pockets between the bonded regions.

17. The device of claim 16, wherein the expansion rings comprise zig-zag expansion rings which each occupy a band of axial extent when radially expanded

18. The device of claim 15, wherein the distributed contact points constitute greater than one half of the surface area of the inner lamina.

19. The device of claim 15, wherein the inner lamina is spaced apart from and not bonded to the outer lamina throughout each band, whereby the expansion rings are free to expand without shearing the lamina.

20. The device of claim 15, wherein the endostent is tubular in shape and the inner lamina loops back over itself to form the cuff and the outer lamina thereby closing an end of the sheath to maintain the member enveloped therein.

21. The device of claim 14, wherein the expandable member comprises a balloon-expandable tube formed of metal expansion mesh material.

22. A method of forming an implantable prosthesis in the form of an endostent assembly for stenting an anatomical passage, such method comprising the steps of:
providing a radially expandable stent member; and
enveloping the stent member by providing an inner sheet of radially-expandable material and folding the sheet back to enclose the stent within a cuff;
wherein the step of enveloping includes joining the inner sheet to the outer sheet over a distributed region in such a way that when the stent member is expanded the sheets do not delaminate.

23. A method of fabricating an implantable prosthesis, comprising;
inserting a tube of PTFE material centrally inside an interior of a stent body;
folding back a portion of the tube over itself in such a way that the tube extends inside and outside the stent body forming a cuff seamlessly and continuously enclosing at least one end thereof within a cocoon of PTFE material; and
coalescing the tube to seal the stent body within the cocoon.

24. The method of claim 23, wherein the step of folding back a portion includes a step of radially expanding the portion prior to the step of folding back a portion.

25. The method of claim 23, further including the step of increasing porosity of the portion of the tube.

26. An implantable prosthetic device in the form of an endostent for insertion into a biological flow passage for expansion to maintain the passage open, comprising:
a radially expandable member shaped and adapted for insertion within the passage and for being expanded radially within the passage to apply outward pressure against walls of the passage when expanded; and
a polymer sheath forming a seamless and continuous cuff over an end of the member and tightly enclosing the expandable member between substantially continuous inside and outside layers of the sheath.

27. An implantable stent assembly for insertion into a biological flow passage for expansion to maintain the passage open, comprising:
a radially expandable perforate stent member having a plurality of apertures, the member shaped and adapted for insertion within the passage and for being expanded radially within the passage to apply outward pressure against walls of the passage when expanded;
an expandable tube extending over an inner wall of the stent member;
an expandable tube extending over an outer wall of the stent member;
wherein the inner tube extends seamlessly and continuously over at least one end of the stent member; and
wherein both tubes are joined to each other through the apertures.
